(19) Europäisches Patentamt
European Patent Office
Office européen des brevets

(11) EP 3 763 786 A1

(12) # EUROPEAN PATENT APPLICATION
published in accordance with Art. 153(4) EPC

(43) Date of publication:
**13.01.2021 Bulletin 2021/02**

(21) Application number: **19763425.6**

(22) Date of filing: **07.03.2019**

(51) Int Cl.:
***C08L 33/02*** *(2006.01)* ***A61K 8/81*** *(2006.01)*
***A61Q 5/02*** *(2006.01)* ***A61Q 11/00*** *(2006.01)*
***A61Q 19/10*** *(2006.01)* ***C08F 2/38*** *(2006.01)*
***C08F 220/06*** *(2006.01)* ***C08F 228/02*** *(2006.01)*
***C08K 3/30*** *(2006.01)* ***C11D 3/37*** *(2006.01)*

(86) International application number:
**PCT/JP2019/009053**

(87) International publication number:
**WO 2019/172365 (12.09.2019 Gazette 2019/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2018 JP 2018041839**

(71) Applicant: **Nippon Shokubai Co., Ltd.**
**Osaka-shi, Osaka 541-0043 (JP)**

(72) Inventors:
- **MATSUMOTO, Rika**
**Suita-shi, Osaka 564-0034 (JP)**
- **SANO, Yuki**
**Suita-shi, Osaka 564-0034 (JP)**

(74) Representative: **Cabinet Beau de Loménie**
**158, rue de l'Université**
**75340 Paris Cedex 07 (FR)**

(54) **(METH)ACRYLIC ACID COPOLYMER-CONTAINING COMPOSITION AND METHOD FOR PRODUCING (METH)ACRYLIC ACID COPOLYMER**

(57) The present invention provides a (meth)acrylic acid copolymer-containing composition which can sufficiently exhibit both stability at low temperatures and a calcium phosphate scale inhibition ability. The present invention relates to a (meth)acrylic acid copolymer-containing composition containing a (meth)acrylic acid copolymer including: a structural unit (a) derived from at least one selected from the group consisting of (meth)acrylic acid and salts thereof; and a structural unit (b) derived from a sulfonic acid monomer (B), the copolymer containing the structural unit (b) in a proportion of 5 to 90 mol% based on 100 mol% of all structural units, and having a weight average molecular weight of 4,500 to 18,000, the composition containing a sulfur-containing inorganic component not incorporated into the copolymer at a concentration of 6,000 ppm or lower in terms of sulfur atoms.

EP 3 763 786 A1

## Description

### TECHNICAL FIELD

The present invention relates to a (meth)acrylic acid copolymer-containing composition and a method of producing a (meth)acrylic acid copolymer. Specifically, the present invention relates to a (meth)acrylic acid copolymer-containing composition and a method of producing a (meth)acrylic acid copolymer, useful for water treatment chemicals.

### BACKGROUND ART

(Meth)acrylic acid copolymers, which are water-soluble polymers, are widely used in various fields. In particular, low-molecular-weight (meth)acrylic acid copolymers are excellent in chelating and dispersing abilities, and are suitable for various applications. Specifically, they are suitably used for dispersants such as inorganic pigments and metal ions; detergent builders; and water treatment chemicals such as corrosion inhibitors and scale inhibitors. For example, in a cooling water system, a boiler water system, seawater desalination equipment, a pulp melting tank, and a black liquor concentration tank, deposits (scale) of calcium carbonate, zinc phosphate, calcium phosphate, zinc hydroxide, magnesium silicate, or the like adhere to their walls. This causes obstacles to operation, such as reduction in thermal efficiency and localized corrosion. To inhibit or remove such scale, (meth)acrylic acid copolymers are used as corrosion inhibitors or scale inhibitors.

As for (meth)acrylic acid copolymers and methods of producing (meth)acrylic acid copolymers, for example, Patent Literature 1 discloses a (meth)acrylic acid copolymer containing a structural unit (a) derived from a (meth)acrylic acid-based monomer (A) having a specific structure and a structural unit (b) derived from a monoethylenically unsaturated monomer (B) copolymerizable with the (meth)acrylic acid-based monomer (A), wherein a sulfonic acid group is present at at least one end of the main chain (the sulfonic acid group may be in the form of an ammonium salt, alkali metal salt, or salt of an organic amine group); the structural unit (b) contains at least a structural unit (b1) derived from a (meth)allyl ether-based monomer (B1) having a specific structure; and the proportions of the structural unit (a) and the structural unit (b1) are respectively 70 to 95% by mole and 5 to 30% by mole.

Patent Literature 2 discloses a method of producing a water-soluble (meth)acrylic acid polymer. The method includes producing a (meth)acrylic acid polymer by solution polymerization of a water-soluble (meth)acrylic acid monomer in which the water-soluble (meth)acrylic acid monomer in such an amount that the polymer concentration in the reaction liquid after polymerization will be 38 to 72% by weight in terms of the monomer, a polymerization initiator, and hypophosphorous acid (salt) are sequentially introduced into an aqueous medium to polymerize the monomer.

Patent Literatures 3 and 4 disclose (meth)acrylic acid copolymers for water treatment chemicals or the like.

### CITATION LIST

- Patent Literature

Patent Literature 1: JP 2002-003536 A
Patent Literature 2: JP H06-263803 A
Patent Literature 3: JP H06-287208 A
Patent Literature 4: JP 2014-511402 T

### SUMMARY OF INVENTION

- Technical Problem

Various (meth)acrylic acid copolymers for water treatment chemicals or the like have been proposed as described above. Still, in compositions containing conventional (meth)acrylic acid copolymers, sodium sulfate tends to be deposited when the compositions are stored at low temperatures, and the compositions are insufficient in calcium phosphate scale inhibition and do not have a sufficiently good balance between stability at low temperatures and a calcium phosphate scale inhibition ability. In particular, since sodium sulfate once deposited in the compositions is difficult to remove, (meth)acrylic acid copolymers have a disadvantage in shipping, storage, and handling.

The present invention has been made in view of such a current state of the art and aims to provide a (meth)acrylic acid copolymer-containing composition which can sufficiently exhibit both stability at low temperatures and a calcium phosphate scale inhibition ability.

- Solution to Problem

The present inventors have conducted various studies on a composition containing a (meth)acrylic acid copolymer and found that a composition containing a sulfur-containing inorganic component not incorporated into a copolymer of the composition at a concentration in terms of sulfur atoms within a certain range can sufficiently inhibit deposition of sodium sulfate even when it is stored at low temperatures and has excellent stability at low temperatures. The present inventors also found that a composition containing a copolymer that contains a certain proportion of a structural unit derived from a sulfonic acid monomer and has a weight average molecular weight within a certain range has an excellent calcium phosphate scale inhibition ability. Thereby, the inventors have arrived at an admirable solution to the above problem, completing the present invention.

That is, the present invention relates to a (meth)acrylic acid copolymer-containing composition containing a (meth)acrylic acid copolymer including:

a structural unit (a) derived from at least one selected from the group consisting of (meth)acrylic acid and salts thereof; and
a structural unit (b) derived from a sulfonic acid monomer (B),
the copolymer containing the structural unit (b) in a proportion of 5 to 90 mol% based on 100 mol% of all structural units, and having a weight average molecular weight of 4,500 to 18,000,
the composition containing a sulfur-containing inorganic component not incorporated into the copolymer at a concentration of 6,000 ppm or lower in terms of sulfur atoms.

Preferably, the copolymer contains the structural unit (b) in a proportion of 10 to 90 mol% based on 100 mol% of all structural units.

Preferably, the copolymer contains the structural unit (a) in a proportion of 10 to 95 mol% based on 100 mol% of all structural units.

Preferably, the copolymer contains a structural unit (e) derived from a monomer (E) other than (meth)acrylic acid (salt) and the sulfonic acid monomer (B) in a proportion of 0 to 20 mol% based on 100 mol% of all structural units.

Preferably, the copolymer has a weight average molecular weight of 4,500 to 17,000.

Preferably, the sulfonic acid monomer (B) is represented by the following formula (1):

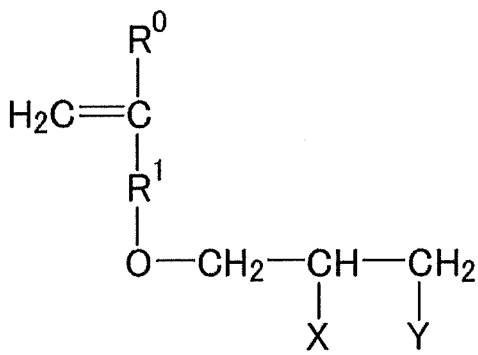

(1)

wherein $R^0$ is a hydrogen atom or a methyl group; $R^1$ is a $CH_2$ group, a $CH_2CH_2$ group, or a direct bond; X and Y are each selected from the group consisting of a hydroxy group and structures represented by the following formulas (2) and (3), with at least one of X or Y being a structure represented by the formula (2) or (3):

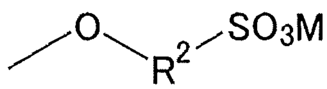

(2)

$-SO_3M$ (3)

wherein $R^2$ is an optionally substituted C1-C10 alkylene group or an optionally substituted C6-C10 arylene group; M in the formulas (2) and (3) is at least one selected from the group consisting of a hydrogen atom, a metal atom, an ammonium group, an organic amine salt, and an organic ammonium group.

Preferably, the copolymer contains a phosphorus-containing group.

Preferably, the copolymer contains at least one structure derived from one selected from the group consisting

of hypophosphorous acid, salts thereof, phosphorous acid, and salts thereof.

Preferably, the composition has a percentage of scale inhibition of 40% or higher determined by a test for calcium phosphate scale inhibition,
the test for calcium phosphate scale inhibition including:

allowing a test solution containing the copolymer at a concentration of 12 mg/L and phosphoric acid ions at a concentration of 9 mg $PO_4^{3-}$/L and having a calcium hardness in terms of calcium carbonate of 500 mg $CaCO_3$/L to stand at 60°C for 24 hours; and
analyzing a concentration of residual phosphoric acid ions to determine a percentage of calcium phosphate scale inhibition using the following formula:

$$\text{Percentage of calcium phosphate scale inhibition (\%)} = 100 \times (R - Q)/(P - Q)$$

wherein P is a concentration (mg/L) of phosphoric acid ions in the test solution at the start of the test (prepared solution); Q is a concentration (mg/L) of residual phosphoric acid ions in a blank solution not containing the copolymer; and R is a concentration (mg/L) of residual phosphoric acid ions.

The present invention also relates to a water treatment chemical containing the (meth)acrylic acid copolymer-containing composition.

The present invention also relates to a cleaning agent containing the (meth)acrylic acid copolymer-containing composition.

The present invention also relates to a method of producing a (meth)acrylic acid copolymer, the method containing:

polymerizing a monomer component containing at least one selected from the group consisting of (meth)acrylic acid and salts thereof and a sulfonic acid monomer (B) in the presence of a phosphorus-containing compound to prepare a copolymer,
the monomer component containing the sulfonic acid monomer (B) in a proportion of 5 to 90 mol% based on 100 mol% of the entire monomer component,
the copolymer having a weight average molecular weight of 4,500 to 18,000.

Preferably, in the production method, the copolymer contains a structural unit (b) in a proportion of 10 to 90 mol% based on 100 mol% of all structural units.

Preferably, in the production method, the copolymer contains the structural unit (a) in a proportion of 10 to 95 mol% based on 100 mol% of all structural units.

Preferably, in the production method, the copolymer contains a structural unit (e) derived from a monomer (E) other than (meth)acrylic acid (salt) and the sulfonic acid monomer (B) in a proportion of 0 to 20 mol% based on 100 mol% of all structural units.

Preferably, in the production method, the copolymer has a weight average molecular weight of 4,500 to 17,000.

Preferably, in the polymerization, the sulfonic acid monomer (B) is continuously added dropwise to a reaction vessel at two or more different drop rates.

Preferably, the phosphorus-containing compound is hypophosphorous acid or a salt thereof.

- Advantageous Effects of Invention

The (meth)acrylic acid copolymer-containing composition of the present invention having the above features can sufficiently exhibit both stability at low temperatures and a calcium phosphate scale inhibition ability, and is thus suitable for water treatment chemicals, for example.

DESCRIPTION OF EMBODIMENTS

The following description is offered to specifically illustrate preferred embodiments of the present invention. It should be noted that the present invention is not limited only to these embodiments, and the embodiments can be appropriately altered within the scope of the present invention. Any combination of two or more of the following preferred embodiments of the present invention is also a preferred embodiment of the present invention. Unless otherwise mentioned, the term "ppm" herein means "ppm by mass".

<(Meth)acrylic acid copolymer-containing composition>

A (meth)acrylic acid copolymer-containing composition of the present invention (hereinafter, also referred to as a composition of the present invention) contains a (meth)acrylic acid copolymer including: a structural unit (a) derived from (meth)acrylic acid (salt); and a structural unit (b) derived from a sulfonic acid monomer (B), the composition containing a sulfur-containing inorganic component not incorporated into the copolymer at a concentration of 6,000 ppm or lower in terms of sulfur atoms. In this composition, deposition of sodium sulfate can be sufficiently inhibited.

The sulfur-containing inorganic component not incorporated into the copolymer is a sulfur-containing inorganic component contained in the composition as byproducts or unreacted materials such as a polymerization initiator and a chain transfer agent, for example.

The concentration of the sulfur-containing inorganic component not incorporated into the copolymer in terms of sulfur atoms is preferably 5,500 ppm or lower, more preferably 5,000 ppm or lower, still more preferably 4,000 ppm or lower, particularly preferably 3,000 ppm or lower.

The concentration of the sulfur-containing inorganic component in terms of sulfur atoms is preferably higher than 0 ppm, more preferably 500 ppm or higher, still more preferably 1,000 ppm or higher, further preferably 1,500 ppm or higher, particularly preferably 2,000 ppm or higher.

A composition containing the sulfur-containing inorganic component at a concentration of 6,000 ppm or lower in terms of sulfur atoms can be obtained, for example, by controlling in the production of the copolymer the amount of a sulfur-containing polymerization initiator and a sulfur-containing chain transfer agent.

The concentration of the sulfur-containing inorganic component not incorporated into the copolymer in terms of sulfur atoms may be measured by the method described in the Examples.

The composition of the present invention preferably has a percentage of scale inhibition of 40% or higher determined by a test for calcium phosphate scale inhibition. The percentage is more preferably 60% or higher, still more preferably 70% or higher, particularly preferably 80% or higher.

In the composition of the present invention, the proportion of the (meth)acrylic acid copolymer based on 100% by mass of the composition is preferably 30 to 70% by mass, more preferably 35 to 60% by mass, still more preferably 40 to 55% by mass.

The composition of the present invention preferably has a pH of 1.5 to 6, more preferably 5 or less. A composition having a pH within the range indicated above is preferred because it has better stability at low temperatures.

<(Meth)acrylic acid copolymer>

The proportion of the structural unit (b) in the (meth)acrylic acid copolymer is 5 to 90 mol% based on 100 mol% of all structural units.

The lower limit of the proportion of the structural unit (b) is preferably 7.5 mol% or higher. With such a proportion, the composition of the present invention has a better calcium phosphate scale inhibition ability. The lower limit of the proportion of the structural unit (b) is more preferably 10 mol% or higher, still more preferably 12.5 mol% or higher, further preferably 15 mol% or higher, further more preferably 17 mol% or higher, still further more preferably 18 mol% or higher, particularly preferably 20 mol% or higher. The upper limit of the proportion of the structural unit (b) is preferably 70 mol% or less, more preferably 60 mol% or less, still more preferably 50 mol% or less, further preferably 40 mol% or less, particularly preferably 30 mol% or less.

The proportion of the structural unit (a) in the (meth)acrylic acid copolymer is preferably 10 to 95 mol% based on 100 mol% of all structural units. With such a proportion, the composition of the present invention has a better calcium trapping ability and a better calcium phosphate scale inhibition ability. The lower limit of the proportion of the structural unit (a) is more preferably 30 mol% or higher, still more preferably 40 mol% or higher, further preferably 50 mol% or higher, particularly preferably 60 mol% or higher. The upper limit of the proportion of the structural unit (a) is more preferably 90 mol% or less, still more preferably 85 mol% or less, further preferably 83mol% or less, further more preferably 82 mol% or less, particularly preferably 80 mol% or less.

The (meth)acrylic acid copolymer may optionally contain a structural unit (e) derived from a monomer (E) other than (meth)acrylic acid (salt) and the sulfonic acid monomer (B). The proportion of the structural unit (e) may be, but not limited to, 0 to 20 mol%, more preferably 0 to 10 mol%, still more preferably 0 to 5 mol%, most preferably 0 mol% based on 100 mol% of all structural units.

Preferably, the (meth)acrylic acid copolymer contains a phosphorus-containing group. With the presence of a phosphorus-containing group, the composition of the present invention can be used as a marker for analysis of the polymer concentration when used for, for example, water treatment chemicals. Further, the composition of the present invention can enhance the heat resistance.

More preferably, the (meth)acrylic acid copolymer contains a structure derived from hypophosphorous acid (salt) and/or a structure derived from phosphorous acid (salt), i.e., a hypophosphorous acid (salt) group and/or a phosphorous acid (salt) group. Still more preferably, the (meth)acrylic acid copolymer contains a hypophosphorous acid (salt) group.

The hypophosphorous acid (salt) group encompasses a hypophosphorous acid group and salts thereof, and the phosphorous acid (salt) group encompasses a phosphorous acid group and salts thereof.

The (meth)acrylic acid copolymer containing a phosphorus-containing group can be obtained by using a phosphorus-containing compound as a chain transfer agent in the production of the copolymer, for example.

For example, when hypophosphorous acid (salt) is used as a chain transfer agent, a hypophosphorous acid (salt) group can be introduced at an end of the main chain of the copolymer.

A structural unit containing a hypophosphorous acid (salt) group at an end of the main chain can be analyzed by $^{31}P$-NMR, for example.

When a compound having multiple reaction sites, such as the hypophosphorous acid (salt), is used as a chain transfer agent in the production of the copolymer, a phosphorus-containing structural unit may be introduced at sites other than the molecular ends of the copolymer (for example, a sodium phosphinate group may be incorporated in the molecule and present at sites other than and in addition to the molecular ends as -P(=O)(ONa)-). Even in this case, the phosphorus-containing structural unit at a molecular end can be analyzed by $^{31}P$-NMR, for example.

The (meth)acrylic acid copolymer has a weight average molecular weight of 4,500 to 18,000. The weight average molecular weight is preferably 4,500 to 17,000, more preferably 16,000 or less, still more preferably 15,000 or less, further preferably 14,000 or less, particularly preferably 13,000 or less.

The weight average molecular weight is more preferably 5,000 or more, still more preferably 5,500 or more, further preferably 6,000 or more, particularly preferably 6,500 or more.

When the copolymer contains the structural unit (b) in a proportion of 20 mol% or higher, even a copolymer having a relatively large weight average molecular weight of 10,000 to 15,000 can further sufficiently exhibit a calcium phosphate scale inhibition ability.

The weight average molecular weight of the copolymer can be measured by the method described in the Examples.

The (meth)acrylic acid copolymer contains the structural unit (a) derived from (meth)acrylic acid (salt). The (meth)acrylic acid (salt) encompasses acrylic acid, salts of acrylic acid, methacrylic acid, and salts of methacrylic acid. Preferred among these are acrylic acid and salts of acrylic acid. One of these substances encompassed by the (meth)acrylic acid (salt) may be used alone, or two or more of these may be used in combination.

The salts of (meth)acrylic acid may be metal salts, ammonium salts, or organic amine salts. Preferred among these are alkali metal salts, with sodium salts being more preferred. One of these salts of (meth)acrylic acid may be used alone or two or more of these may be used in combination.

The (meth)acrylic acid copolymer contains the structural unit (b) derived from a sulfonic acid monomer (B). The sulfonic acid monomer (B) may be any monomer containing a sulfonic acid (salt) group and an ethylenically unsaturated hydrocarbon group. Examples thereof include (poly)alkylene glycol-containing unsaturated sulfonic acids such as 3-(meth)allyloxy-2-hydroxypropanesulfonic acid, 2-(meth)allyloxyethylenesulfonic acid, 2-acrylamido-2-methylpropanesulfonic acid, p-styrenesulfonic acid, $\alpha$-methyl-p-styrenesulfonic acid, vinylsulfonic acid, vinylsulfamic acid, (meth)allylsulfonic acid, isoprenesulfonic acid, 4-(allyloxy)benzenesulfonic acid, 1-methyl-2-propene-1-sulfonic acid, 1,1-dimethyl-2-propene-1-sulfonic acid, 3-butene-1-sulfonic acid, 1-butene-3-sulfonic acid, 2-acrylamido-1-methylpropanesulfonic acid, 2-acrylamidopropanesulfonic acid, 2-acrylamido-n-butanesulfonic acid, 2-acrylamido-2-phenylpropanesulfonic acid, 2-((meth)acryloyloxy)ethanesulfonic acid, 2-(meth)allyloxyethylenesulfonic acid, and a monomer represented by the following formula (4):

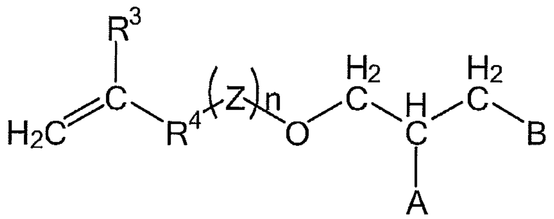

(4)

wherein $R^3$ is a hydrogen atom or a methyl group; $R^4$ is a $CH_2$ group, a $CH_2CH_2$ group, or a direct bond; Zs are the same as or different from each other and are each a C2-C20 oxyalkylene group; n is the average number of moles of oxyalkylene groups added and is 1 to 300; A and B are each selected from the group consisting of a hydroxy group and structures represented by the following formulas (5) and (6), with at least one of A or B being a structure represented by the formula (5) or (6):

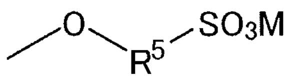 (5)

$-SO_3M$ (6)

wherein $R^5$ is an optionally substituted C1-C10 alkylene group or an optionally substituted C6-C10 arylene group; and M in the formulas (5) and (6) is at least one selected from the group consisting of a hydrogen atom, a metal atom, an ammonium group, an organic amine salt, and an organic ammonium group) and salts of these.

In the formula (4), Zs are "the same as or different from each other" and are each a C2-C20 oxyalkylene group. This means that n oxyalkylene groups for Zs in the polyalkylene glycol may all be the same as or different from each other.

The oxyalkylene group for Z is an alkylene oxide adduct, and examples of the alkylene oxide include C2-C8 alkylene oxides such as ethylene oxide, propylene oxide, butylene oxide, isobutylene oxide, 1-butene oxide, 2-butene oxide, and styrene oxide. Preferred are C2-C4 alkylene oxides, with ethylene oxide and propylene oxide being more preferred.

To balance hydrophilicity and hydrophobicity, the oxyalkylene groups in the polyalkylene glycol preferably essentially include oxyethylene groups. More preferably, the oxyethylene groups constitute 50 mol% or more of the oxyalkylene groups, still more preferably constitute 90 mol% or more of the oxyalkylene groups.

In the formula (4), n is the average number of moles of oxyalkylene groups added, and n is 1 to 300, preferably 2 to 100, more preferably 3 to 55.

Preferably, at least one of A or B is a structure represented by the formula (6).

More preferably, one of A and B is a hydroxy group and the other is a sulfonic acid (salt) group. Still more preferably, A is a hydroxy group and B is a sulfonic acid (salt) group.

The optionally substituted C1-C10 alkylene group and optionally substituted C6-C10 arylene group in the formula (5) are the same as the below-described optionally substituted C1-C10 alkylene group and optionally substituted C6-C10 arylene group in the formula (2), respectively.

The salts of the sulfonic acid group may be, for example, the same as the salts exemplified for the salts of (meth)acrylic acid.

Preferably, the sulfonic acid monomer (B) is represented by the following formula (1):

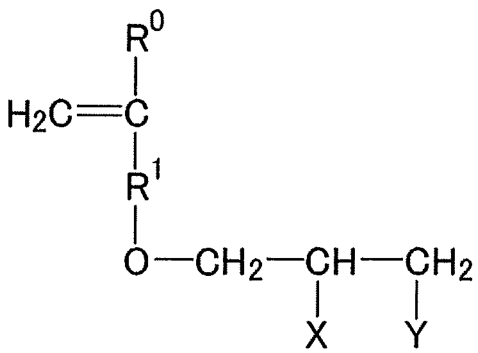 (1)

wherein $R^0$ is a hydrogen atom or a methyl group; $R^1$ is a $CH_2$ group, a $CH_2CH_2$ group, or a direct bond; X and Y are each selected from the group consisting of a hydroxy group and structures represented by the following formulas (2) and (3), with at least one of X or Y being a structure represented by the formula (2) or (3):

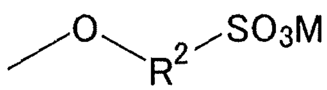 (2)

$-SO_3M$ (3)

wherein $R^2$ is an optionally substituted C1-C10 alkylene group or an optionally substituted C6-C10 arylene group; M in the formulas (2) and (3) is at least one selected from the group consisting of a hydrogen atom, a metal atom, an ammonium group, an organic amine salt, and an organic ammonium group. In other words, the copolymer preferably contains a structural unit derived from a monomer represented by the formula (1).

$R^0$ is preferably a hydrogen atom.

$R^1$ is preferably a $CH_2$ group. When $R^1$ is a $CH_2$ group, the effects of the present invention can be more effectively achieved.

In the formula (2), the C1-C10 alkylene group and the C6-C10 arylene group may each optionally contain a substituent. Examples of the substituent include alkenyl, alkoxy, hydroxy, acyl, ether, amide, ester, and ketone groups.

The number of carbon atoms of each of the alkylene group and the arylene group refers to the number of carbon atoms including the carbon atoms of a substituent.

Examples of the alkylene group include methylene, methylmethylene, ethylene, n-propylene, isopropylene, n-butylene, isobutylene, sec-butylene, tert-butylene, n-pentylene, isopentylene, neopentylene, hexylene, heptylene, octylene, nonylene, and decylene groups.

The number of carbon atoms of the alkylene group is preferably 1 to 8, more preferably 1 to 4.

Examples of the arylene group include a group obtained by abstracting one hydrogen atom from an aryl group such as a pheny, benzyl, 1-phenylethyl, 2-phenylethyl, 3-phenylpropyl, 4-phenylbutyl, styryl (Ph-CH=C-), or cinnamyl (Ph-CH=$CHCH_2$-) group.

The number of carbon atoms of the arylene group is preferably 6 to 8, more preferably 6 or 7.

$R^2$ is preferably an optionally substituted C1-C10 alkylene group.

Preferably, at least one of X or Y is a structure represented by the formula (3).

More preferably, one of X and Y is a hydroxy group and the other is a sulfonic acid (salt) group. Still more preferably, X is a hydroxy group and Y is a sulfonic acid (salt) group.

The sulfonic acid monomer (B) is more preferably 3-(meth)allyloxy-2-hydroxy-1-propanesulfonic acid or a salt thereof. Since 3-(meth)allyloxy-2-hydroxy-1-propanesulfonic acid is less susceptible to hydrolysis and is highly stable, it is environmentally friendly and highly safe. Further, 3-(meth)allyloxy-2-hydroxy-1-propanesulfonic acid is more economically efficient than p-styrenesulfonic acid, for example.

The (meth)acrylic acid copolymer may further contain a structural unit (e) derived from a monomer (E) other than (meth)acrylic acid (salt) and the sulfonic acid monomer (B). Examples of the monomer (E) include carboxyl group-containing monomers (excluding (meth) acrylic acid) such as maleic acid, fumaric acid, itaconic acid, crotonic acid, and 2-methylene glutaric acid, and salts thereof; hydroxy group-containing alkyl (meth)acrylates such as 2-hydroxyethyl (meth)acrylate, 2-hydroxypropyl (meth)acrylate, 3-hydroxypropyl (meth)acrylate, 2-hydroxybutyl (meth)acrylate, 4-hydroxybutyl (meth)acrylate, and $\alpha$-hydroxymethylethyl (meth)acrylate; alkyl (meth)acrylates which are C1-C18 alkyl esters of (meth)acrylic acid, such as methyl (meth)acrylate, ethyl (meth)acrylate, butyl (meth)acrylate, cyclohexyl (meth)acrylate, and lauryl (meth)acrylate; amino group-containing acrylates such as dimethylaminoethyl (meth)acrylate and quaternized compounds thereof; amide group-containing monomers such as (meth)acrylamide, dimethylacrylamide, and isopropylacrylamide; vinyl esters such as vinyl acetate; alkenes such as ethylene and propylene; aromatic vinyl monomers such as styrene; maleimide and maleimide derivatives such as phenylmaleimide and cyclohexylmaleimide; nitrile group-containing vinyl monomers such as (meth)acrylonitrile; phosphonic acid group-containing monomers such as vinylphosphonic acid and (meth)allylphosphonic acid; aldehyde group-containing vinyl monomers such as (meth)acrolein; alkyl vinyl ethers such as methyl vinyl ether, ethyl vinyl ether, and butyl vinyl ether; functional group-containing monomers other than the aforementioned monomers such as vinyl chloride, vinylidene chloride, allyl alcohol, and vinylpyrrolidone; and polyalkylene glycol chain-containing monomers such as polyalkylene glycol (meth)acrylate, monoalkoxy polyalkylene glycol (meth)acrylate, and a monomer having a structure in which 1 to 300 mol of an alkylene oxide is added to an unsaturated alcohol such as vinyl alcohol, (meth)allyl alcohol, or isoprenol. These monomers (E) may be used alone, or two or more of these may be used in combination.

<Method of producing (meth)acrylic acid copolymer>

The present invention also relates to a method of producing a (meth)acrylic acid copolymer, the method including: polymerizing a monomer component containing (meth)acrylic acid (salt) and a sulfonic acid monomer (B) in the presence of a phosphorus-containing compound to prepare a copolymer, the monomer component containing the sulfonic acid monomer (B) in a proportion of 5 to 90 mol% based on 100 mol% of the entire monomer component, the copolymer having a weight average molecular weight of 4,500 to 18,000.

Specific examples and preferred examples of the monomer component and preferred proportions of the monomers are as described above.

The (meth)acrylic acid copolymer-containing composition of the present invention may be produced by any method, and is preferably produced by the method of producing a (meth)acrylic acid copolymer of the present invention.

In the polymerization, a phosphorus-containing compound is preferably used as a chain transfer agent.

Preferred examples of the phosphorus-containing compound include hypophosphorous acid (salt) (including hydrates thereof) such as hypophosphorous acid and sodium hypophosphite and phosphorous acid (salt) such as phosphorous acid and sodium phosphite. The presence of any of these chain transfer agents in polymerization of the monomer component can introduce a phosphorus-containing group to an end of the main chain of the copolymer.

Preferred among these is hypophosphorous acid (salt).

In the production of the copolymer of the present invention, the amount of the phosphorus-containing compound per mole of the monomer component (all monomers) is preferably 0.01 g or more and 10 g or less, more preferably 0.1 g or more and 8 g or less, still more preferably 6 g or less, further preferably 5 g or less, particularly preferably 4 g or less.

In the polymerization, a different chain transfer agent other than and in addition to the phosphorus-containing compound may be used. Examples of the different chain transfer agent include thiol chain transfer agents such as mercaptoethanol and mercaptopropionic acid; halides such as carbon tetrachloride and methylene chloride; secondary alcohols such as isopropyl alcohol and glycerol; sulfurous acid (salt) such as sodium sulfite; bisulfurous acid (salt) such as sodium bisulfite; dithionous acid (salt) such as sodium dithionite; and metabisulfurous acid (salt) such as potassium metabisulfite. These different chain transfer agents may be used alone, or two or more of these may be used in combination.

The amount of the different chain transfer agent used is preferably 5 g or less, more preferably 3 g or less, still more preferably 1 g or less, most preferably 0 g, per mole of the monomer component (all monomers).

In the polymerization, the polymerization of the monomer component may be started by any method, such as addition of a polymerization initiator, UV irradiation, heat application, or light irradiation in the presence of a photo initiator.

In the polymerization, a polymerization initiator is preferably used.

Preferred examples of the polymerization initiator include hydrogen peroxide; persulfates such as sodium persulfate, potassium persulfate, and ammonium persulfate; azo compounds such as dimethyl-2,2'-azobis(2-methylpropionate), 2,2'-azobis(isobutyronitrile), 2,2'-azobis(2-methylpropionamidine) dihydrochloride (2,2'-azobis-2-amidinopropane dihydrochloride), 2,2'-azobis[N-(2-carboxyethyl)-2-methylpropionamidine] hydrate, 2,2'-azobis[2-(2-imidazolin-2-yl)propane], 2,2'-azobis[2-(2-imidazolin-2-yl)propane] dihydrochloride, and 2,2'-azobis(1-imino-1-pyrrolidino-2-methylpropane) dihydrochloride; organic peroxides such as benzoyl peroxide, lauroyl peroxide, peracetic acid, di-t-butyl peroxide, and cumene hydroperoxide; and redox initiators that generate radicals by combining an oxidizing agent and a reducing agent, such as a combination of ascorbic acid and hydrogen peroxide and a combination of a persulfate and a metal salt. Preferred among these polymerization initiators are hydrogen peroxide, persulfates, and azo compounds, with persulfates being more preferred, because they tend to reduce the amount of residual monomers. These polymerization initiators may be used alone, or two or more of these may be used in the form of a mixture.

The amount of the polymerization initiator used per mole of the monomer component (all monomers) is preferably 0.1 g or more and 10 g or less, more preferably 0.2 g or more and 8 g or less, still more preferably 0.5 g or more and 7 g or less.

When a persulfate in an amount within the range indicated above is used as a polymerization initiator, the composition of the present invention can have a concentration of a sulfur-containing inorganic component not incorporated into the copolymer in terms of sulfur atoms within a more suitable range.

When a solvent is used in the polymerization, it is preferably an aqueous solvent. Examples of the aqueous solvent include water; alcohols such as methyl alcohol, ethyl alcohol, isopropyl alcohol (2-propanol), n-butyl alcohol, and diethylene glycol; and glycol, glycerol, and polyethylene glycol. Preferred is water.

To enhance the solubility of the monomers in solvents, any appropriate organic solvent may be suitably added as needed as long as it has no adverse effects on the polymerization. Examples the organic solvent include lower alcohols such as methanol, ethanol, and isopropyl alcohol; lower ketones such as acetone, methyl ethyl ketone, and diethyl ketone; ethers such dimethyl ether, diethyl ether, and dioxane; and amides such as dimethyl formaldehyde. These solvents may be used alone, or two or more of these may be used in combination.

The amount of the solvent used is preferably 40 to 300% by mass per 100% by mass of the monomer component.

In the polymerization in the production method of the present invention, the materials may be added in any way, and they may be added to a reaction vessel in one portion or successively. Preferably, they are successively added. When the materials are successively added, specifically, they may be added continuously (e.g., added dropwise over a certain period of time), intermittently (e.g., added in portions), or by a combination of these. More preferably, the materials are continuously added.

The sulfonic acid monomer (B) is preferably continuously added dropwise to a reaction vessel, more preferably continuously added dropwise to a reaction vessel at two or more different drop rates. That is, in a preferred embodiment of the present invention, in the polymerization, the sulfonic acid monomer (B) is continuously added dropwise to a reaction vessel at two or more different drop rates.

Still more preferably, the sulfonic acid monomer (B) is added dropwise to a reaction vessel at two different drop rates. The dropwise addition of the sulfonic acid monomer (B) to a reaction vessel at two different drop rates can provide a copolymer having a more uniform composition. Particularly preferably, 10 to 90% by mass of 100% by mass of the sulfonic acid monomer (B) is first added dropwise at a constant drop rate (first step), and then, the remaining sulfonic acid monomer (B) is added dropwise at a lower and constant drop rate (second step). The drop rate in the second step is preferably 20% to 70%, more preferably 30% to 60% of the drop rate in the first step, which is taken as 100%.

The polymerization initiator is preferably continuously added dropwise to a reaction vessel. The continuous

dropwise addition of the polymerization initiator to a reaction vessel can reduce the amount of residual monomers. More preferably, the polymerization initiator is continuously added dropwise to a reaction vessel at two or more different drop rates, still more preferably at two different drop rates. Particularly preferably, 5 to 80% by mass of 100% by mass of the polymerization initiator is first added dropwise at a constant drop rate (first step), and then, the remaining polymerization initiator is added dropwise at a higher and constant drop rate (second step). The drop rate in the second step is preferably 105% to 250%, more preferably 110% to 230% of the drop rate in the first step, which is taken as 100%.

The (meth)acrylic acid (salt) and the phosphorus-containing compound are each preferably continuously added dropwise to a reaction vessel. More preferably, the whole amount of each component is added dropwise at a constant rate.

In the polymerization, the pH is preferably 1.5 to 6, more preferably 5 or less. The pH within the range indicated above is preferred because the reaction efficiently proceeds.

Examples of a pH adjuster include alkali metal hydroxides such as sodium hydroxide and potassium hydroxide, alkaline-earth metal hydroxides such as calcium hydroxide and magnesium hydroxide, ammonia, and salts of organic amines such as monoethanolamine and triethanolamine. These may be used alone, or two or more of these may be used in combination. Preferred among these are alkali metal hydroxides such as sodium hydroxide and potassium hydroxide, with sodium hydroxide being more preferred. The degree of neutralization is preferably such that 0 to 75 mol% of the acid groups in the polymer is neutralized.

In the polymerization, the polymerization temperature may be any temperature. A polymerization temperature within 50°C to 150°C is preferred because a higher polymerization ratio can be achieved. The polymerization temperature is more preferably 70°C to 120°C.

In the polymerization, the duration of the reaction may be appropriately set depending on the reaction temperature and the types (properties), combinations, and amounts of the monomer component, polymerization initiators, and solvents, for example, so that the polymerization reaction is to be completed.

The method of producing the copolymer preferably includes aging the copolymer after the polymerization reaction. The aging can reduce the amount of residual monomers. The temperature in the aging may be any temperature, and is preferably 50°C to 150°C. More preferably, the polymerization temperature is maintained.

In the aging, the duration of aging may not be limited, and is preferably 10 minutes to 5 hours, more preferably 15 minutes to 3 hours.

<Applications of (meth)acrylic acid copolymer-containing composition>

The (meth)acrylic acid copolymer-containing composition of the present invention may be used for coagulants, flocculants, printing inks, adhesives, soil conditioners (soil reforming agents), flame retardants, skin care products, hair care products, additives for shampoo, hair sprays, soaps, and cosmetics, anion exchange resins, dye mordants and aids for fibers and photo films, pigment spreading agents used in papermaking, paper strengthening agents, emulsifiers, antiseptic agents, softening agents for fabrics and paper, additives for lubricants, water treatment chemicals, fiber treatment agents, dispersants, additives for detergents, scale inhibitors, sequestrants, thickeners, various binders, and emulsifiers.

<Water treatment chemicals>

The (meth)acrylic acid copolymer-containing composition of the present invention is preferably used for water treatment chemicals. The present invention also encompasses a method of using the (meth)acrylic acid copolymer-containing composition as a water treatment chemical.

The present invention also relates to a water treatment chemical containing the (meth)acrylic acid copolymer-containing composition.

The water treatment chemical may optionally contain other additives such as polymerized phosphates, phosphonates, anticorrosives, slime control agents, chelating agents, and pH adjusters, if needed.

The water treatment chemical is useful for inhibition of scale in a cooling water circulation system, a boiler water circulation system, a seawater desalination plant, a pulp digester, and a black liquor concentrating kettle, for example. The water treatment chemical may optionally contain an appropriate water-soluble polymer as long as the properties and the effects are not affected.

<Detergent or cleaning agent>

The (meth)acrylic acid copolymer-containing composition of the present invention may be used for detergents and cleaning agents. The present invention also encompasses a method of using the (meth)acrylic acid copolymer-containing composition as a detergent or cleaning agent.

The present invention also relates to a detergent or cleaning agent containing the (meth)acrylic acid copolymer-containing composition.

The (meth)acrylic acid copolymer-containing composition may be used as a detergent additive such as a detergent builder in various detergents for clothing, dishes, household, hair, body, toothpaste, and automobiles, for example.

The present invention also relates to a detergent composition containing the (meth)acrylic acid copolymer-containing composition and a detergent additive other than the compound.

The detergent composition contains the (meth)acrylic acid copolymer in a proportion of preferably 0.1 to 40% by mass, more preferably 0.3 to 30% by mass, still more preferably 0.5 to 20% by mass based on 100% by mass of the composition.

The detergent additive other than the (meth)acrylic acid copolymer-containing composition of the present invention may be any surfactant or any additive commonly used in detergents. The detergent additive may be selected by appropriately referring to common knowledge in the detergent field.

The detergent composition may be a powder detergent composition or liquid detergent composition.

The surfactant is preferably one or more surfactants selected from the group consisting of anionic surfactants, nonionic surfactants, cationic surfactants, and amphoteric surfactants.

Suitable examples of the anionic surfactants include alkylbenzene sulfonates, alkyl ether sulfates, alkenyl ether sulfates, alkyl sulfates, alkenyl sulfates, α-olefin sulfonates, α-sulfonated fatty acids and esters thereof, alkane sulfonates, saturated fatty acid salts, unsaturated fatty acid salts, alkyl ether carboxylates, alkenyl ether carboxylates, amino acid-based surfactants, N-acylamino acid-based surfactants, alkyl phosphoric acid esters and salts thereof, and alkenyl phosphoric acid esters and salts thereof. The alkyl group or alkenyl group of any of these anionic surfactants may contain an alkyl group such as a methyl group as a branch.

Suitable examples of the nonionic surfactants include polyoxyalkylene alkyl ethers, polyoxyalkylene alkenyl ethers, polyoxyethylene alkylphenyl ethers, higher fatty acid alkanolamides and adducts thereof with an alkylene oxide, sucrose fatty acid esters, alkyl glycoxides, fatty acid glycerol monoesters, and alkylamine oxides. The alkyl group or alkenyl group of any of these nonionic surfactants may contain an alkyl group such as a methyl group as a branch.

Suitable examples of the cationic surfactants include quaternary ammonium salts. Suitable examples of the amphoteric surfactants include carboxyl-type amphoteric surfactants and sulfobetaine-type amphoteric surfactants. The alkyl group or alkenyl group of any of these cationic surfactants and amphoteric surfactants may contain an alkyl group such as a methyl group as a branch.

The surfactant is typically present in the detergent composition in a proportion of 10 to 60% by mass, preferably 15 to 50% by mass, more preferably 20 to 45% by mass, particularly preferably 25 to 40% by mass based on the whole amount of the detergent composition. A detergent composition containing too small an amount of the surfactant may fail to exhibit sufficient detergency, whereas a detergent composition containing too large an amount of the surfactant may be disadvantageous in terms of cost.

## EXAMPLES

The present invention is described in further detail below with reference to examples, but the present invention is not limited to these examples. It should be noted that the terms "part(s)" and "%" refer to "part(s) by mass" and "% by mass", respectively, unless otherwise stated.

Measurement of the weight average molecular weight and number average molecular weight of the copolymer and evaluation of the properties of the copolymer were performed according to the following methods.

<Conditions for measurement of weight average molecular weight and number average molecular weight>

The weight average molecular weight and number average molecular weight of the copolymer were measured by gel permeation chromatography under the following conditions.

Device: HLC-8320 GPC available from Tosoh Corporation
Detector: RI
Column: Shodex Asahipak GF-1G, GF-710-HQ, and GF-310-HQ available from Showa Denko K. K., connected in this order from the sample inlet
Column temperature: 40°C
Flow rate: 0.5 ml/min
Standard substance for calibration curve: sodium polyacrylate available from American Polymer Standards (there are seven different peak top molecular weights (Mps) within 900 to 589,700), sodium propionate (Mp = 94)
Calibration curve: cubic equation based on the Mp values and elution times of the standard
Eluent: 0.1 N aqueous sodium acetate solution

<Measurement of monomer amount>

The amount of unreacted monomers in the polymer was measured by high-performance liquid chromatography under the following conditions.

Conditions for liquid chromatography

Device: Alliance e2695 Separations Module available from Waters
Detector: Photodiode Array Detector 2998 available from Waters, UV wavelength: 200 nm
Column: Shodex RSpak DE-413L available from Showa Denko K. K.
Column temperature: 40°C
Flow rate: 1.0 ml/min
Calibration curve: calibration curve is prepared using each monomer
Eluent: 0.1% aqueous phosphoric acid solution

<Calculation of amount of structural unit derived from monomer in copolymer>

The analyzed values of the proportions of the structural units derived from the monomers in the obtained copolymer were calculated based on the measurement of the amounts of unreacted monomers on the assumption that all monomers consumed in the polymerization reaction were converted into the copolymer.

<Measurement of solid content>

A copolymer prepared by adding 2.0 g of water to 1.2 g of the copolymer composition was dried in an oven heated at 120°C for two hours. The solid content (%) and the volatile content (%) were calculated from the difference in mass before and after drying.

<Measurement of pH>

The pH of the copolymer composition was measured under the following conditions.
Device: pH METER D-52 available from HORIBA
Electrode: glass electrode
Temperature: 25°C

<Measurement of concentration of sulfur-containing inorganic component not incorporated into copolymer in terms of sulfur atoms>

The concentration S of a sulfur-containing inorganic component not incorporated into the copolymer in terms of sulfur atoms was calculated using the following equation:

$$S = a \times (32.1/96.1) + b \times (32.1/80.1)$$

where a is a sulfate ion concentration and b is a sulfite ion concentration, a and b being quantified by ion chromatography under the following conditions.

Conditions of ion chromatography analysis

Device: IC-2010 available from Tosoh Corporation
Detector: conductivity detector
Column: Shodex IC SI-90G and SI-90 4E available from Showa Denko K. K., connected in this order from the sample inlet
Column temperature: 25°C
Flow rate: 1.2 ml/min
Standard substance: sodium sulfate, sodium sulfite
Eluent: 1 mM sodium carbonate + 4 mM sodium hydrogen carbonate + 5% acetone solution

<Measurement of percentage of calcium phosphate scale inhibition (inhibition ability)>

A borate buffer solution (concentration of boric acid (sodium): 50 mM, concentration of sodium chloride: 8.3 mM, pH = 8.6), an aqueous solution of calcium chloride (calcium hardness (in terms of calcium carbonate, the same applies hereinafter) = 2,500 mg $CaCO^3$/L), and a phosphate buffer (aqueous solution of disodium hydrogen phosphate, concentration of phosphoric acid ions: 100 mg/L) were prepared using commercially available regents and deionized water. The (meth)acrylic acid copolymer-containing compositions obtained in the examples and comparative examples were each diluted with deionized water so that solutions of the respective compositions each having a solid content of 100 mg/L were prepared.

To a 225-mL lidded glass vessel were added deionized water, 10.0 g of the borate buffer solution, 20.0 g of the aqueous solution of calcium chloride, 12.0 g of a solution of each composition, and 9.0 g of the phosphate buffer in the stated order. Thereby, 100 mL of a test solution was prepared which had a pH of 8.6, a concentration of the composition in terms of solid content of 12 mg/L, a calcium hardness of 500 mg $CaCO^3$/L, and a concentration of phosphoric acid ions of 9 mg $PO_4^{3-}$/L. Separately, a blank test solution containing deionized water instead of the solution of each composition was prepared as a blank.

Each test solution was sealed and heated in a water bath at 60°C for 24 hours. The test solution was filtered through a filter paper having a pore size of 0.45 μm. The concentration of residual phosphoric acid ions in the filtrate was analyzed.

The percentage of calcium phosphate scale inhibition was determined using the following equation (2):

$$\text{Percentage of calcium phosphate scale inhibition (\%)} = 100 \times (R - Q)/(P - Q) \quad (2)$$

wherein P is a concentration (mg/L) of phosphoric acid ions in the test solution at the start of the test (prepared solution); Q is a concentration (mg/L) of residual phosphoric acid ions in the blank (not containing any of the copolymers); and R is a concentration (mg/L) of residual phosphoric acid ions.

<Measurement of chelating ability (calcium ion trapping ability)>

A 100-cc beaker was charged with 50 g of a 0.001 mol/L aqueous solution of calcium chloride, and the copolymer in an amount of 10 mg in terms of solid content was added thereto. Then, the pH of the aqueous solution was controlled to 9 to 11 with dilute sodium hydroxide. Thereafter, under stirring, 1 ml of a 4 mol/L aqueous solution of potassium chloride was added as a stabilizer for calcium ion electrodes.

The amount of free calcium ions was measured using an ion analyzer (Model EA920 available from Orion Corporation) and a calcium ion electrode (Model 93-20 available from Orion Corporation), and the amount of calcium ions (in milligrams) in terms of calcium carbonate chelated per gram of the copolymer (calcium ion trapping ability which is a kind of chelating ability) was calculated. The calcium ion trapping ability was expressed in "mg $CaCO_3$/g".

<Test for stability at low temperatures>

A 10-cc vial was charged with 5 g of an aqueous solution of the polymer, the temperature was controlled to 5°C, and a microspatula tip of sodium sulfate powder was added. The solution was allowed to stand at 5°C for 24 hours, and the amount of sodium sulfate deposits was visually observed. The stability at low temperatures was evaluated from the volume of the deposits relative to the whole amount of the solution based on the following criteria.
Volume of deposits of 0% to 40%: Good
Volume of deposits of 41% to 70%: Fair
Volume of deposits of 71% to 100%: Poor

<Example 1>

A 2.5-L stainless steel separable flask equipped with a stirrer, a reflux condenser, and a thermometer was initially charged with 296.3 g of pure water, and the contents were heated to the boiling point under stirring. Subsequently, under stirring, the following components were each separately added dropwise into the system under reflux conditions at the boiling point: 496.5 g of a 80% aqueous solution of acrylic acid (hereinafter, abbreviated as 80% AA), 660 g of a 40% aqueous solution of sodium 3-allyloxy-2-hydroxy-1-propanesulfonate (hereinafter, abbreviated as 40% HAPS), 26.9 g of a 45% aqueous solution of sodium hypophosphite (hereinafter, abbreviated as 45% SHP), and 179.4 g of a 15% aqueous solution of sodium persulfate (hereinafter, abbreviated as 15% NaPS). The dropping times were 180

minutes for 80% AA, 140 minutes for 40% HAPS, 180 minutes for 45% SHP, and 200 minutes for 15% NaPS. As for 40% HAPS, 220 g thereof was added dropwise continuously at a constant drop rate from 0 to 30 minutes, and the remaining 440 g thereof was added dropwise continuously at a constant drop rate from 30 to 140 minutes. As for 15% NaPS, 85.2 g thereof was added dropwise continuously at a constant drop rate from 0 to 130 minutes, and the remaining 94.2 g thereof was added dropwise continuously at a constant drop rate from 130 to 200 minutes. As for 80% AA and 45% SHP, they were each added dropwise continuously while the drop rate was kept constant over a period of dropwise addition. After completion of the dropwise addition of all the components, the reflux conditions at the boiling point were maintained for 30 minutes to complete the polymerization. Thus, an aqueous solution of a polymer (1) having a solid concentration of 45.9% and a pH of 3.4 was obtained.

<Example 2>

Reaction was carried out as in Example 1 except that the amount of initially fed pure water was changed to 231.2 g, 80% AA to 407.9 g, 40% HAPS to 780 g, 45% SHP to 42.4 g, and 15% NaPS to 159 g; that 260 g of 40% HAPS was added dropwise from 0 to 30 minutes, and the remaining 520 g thereof was added dropwise from 30 to 140 minutes; and that 75.5 g of 15% NaPS was added dropwise from 0 to 130 minutes, and the remaining 83.5 g thereof was added dropwise from 130 to 200 minutes. Thus, an aqueous solution of a polymer (2) having a solid concentration of 46.2% and a pH of 3.6 was obtained.

<Example 3>

Reaction was carried out as in Example 1 except that the amount of initially fed pure water was changed to 247.6 g, 80% AA to 430 g, 40% HAPS to 734.4 g, 45% SHP to 24.5 g, and 15% NaPS to 163.4 g; that 326.4 g of 40% HAPS was added dropwise from 0 to 30 minutes, and the remaining 408 g thereof was added dropwise from 30 to 155 minutes; and that 77.6 g of 15% NaPS was added dropwise from 0 to 130 minutes, and the remaining 85.8 g thereof was added dropwise from 130 to 200 minutes. Thus, an aqueous solution of a polymer (3) having a solid concentration of 46.2% and a pH of 3.6 was obtained.

<Example 4>

Reaction was carried out as in Example 1 except that the amount of initially fed pure water was changed to 221.8 g, 80% AA to 360 g, 40% HAPS to 614.9 g, 45% SHP to 11.4 g, and 15% NaPS to 136.8 g; that 273.3 g of 40% HAPS was added dropwise from 0 to 40 minutes, and the remaining 341.6 g thereof was added dropwise from 40 to 140 minutes; and that 65 g of 15% NaPS was added dropwise from 0 to 130 minutes, and the remaining 71.8 g thereof was added dropwise from 130 to 200 minutes. Thus, an aqueous solution of a polymer (4) having a solid concentration of 45.5% and a pH of 3.4 was obtained.

<Example 5>

Reaction was carried out as in Example 1 except that the amount of initially fed pure water was changed to 183.6 g, 80% AA to 324 g, 40% HAPS to 840.8 g, 45% SHP to 45.7 g, and 15% NaPS to 120 g; that 420.4 g of 40% HAPS was added dropwise from 0 to 20 minutes, and the remaining 420.4 g thereof was added dropwise from 20 to 60 minutes; that 72 g of 15% NaPS was added dropwise from 0 to 130 minutes, and the remaining 48 g thereof was added dropwise from 130 to 200 minutes; and that the polymerization temperature and the aging temperature were changed to 87°C. Thus, an aqueous solution of a polymer (5) having a solid concentration of 46.8% and a pH of 3.9 was obtained.

<Example 6>

Reaction was carried out as in Example 1 except that the amount of initially fed pure water was changed to 453.8 g, 80% AA to 636.6 g, 40% HAPS to 312.6 g, 45% SHP to 95.2 g, and 15% NaPS to 102.0 g; that 138.9 g of 40% HAPS was added dropwise from 0 to 30 minutes, and the remaining 173.7 g thereof was added dropwise from 30 to 140 minutes; and that 43.6 g of 15% NaPS was added dropwise from 0 to 130 minutes, and the remaining 58.4 g thereof was added dropwise from 130 to 200 minutes. Thus, an aqueous solution of a polymer (6) having a solid concentration of 45.7% and a pH of 2.8 was obtained.

<Example 7>

Reaction was carried out as in Example 1 except that the amount of initially fed pure water was changed to

427.4 g, 80% AA to 577.2 g, 40% HAPS to 432.0 g, 45% SHP to 67.3 g, and 15% NaPS to 96.1 g; that 192.0 g of 40% HAPS was added dropwise from 0 to 30 minutes, and the remaining 240.0 g thereof was added dropwise from 30 to 140 minutes; and that 41.1 g of 15% NaPS was added dropwise from 0 to 130 minutes, and the remaining 55.0 g thereof was added dropwise from 130 to 200 minutes. Thus, an aqueous solution of a polymer (7) having a solid concentration of 45.4% and a pH of 3.1 was obtained.

<Example 8>

Reaction was carried out as in Example 1 except that the amount of initially fed pure water was changed to 402.5 g, 80% AA to 544.1 g, 40% HAPS to 536.3 g, 45% SHP to 23.4 g, and 15% NaPS to 93.7 g; that 238.4 g of 40% HAPS was added dropwise from 0 to 30 minutes, and the remaining 297.9 g thereof was added dropwise from 30 to 140 minutes; and that 40.1 g of 15% NaPS was added dropwise from 0 to 130 minutes, and the remaining 53.6 g thereof was added dropwise from 130 to 200 minutes. Thus, an aqueous solution of a polymer (8) having a solid concentration of 45.5% and a pH of 3.3 was obtained.

<Example 9>

Reaction was carried out as in Example 1 except that the amount of initially fed pure water was changed to 278.6 g, 80% AA to 405.1 g, 40% HAPS to 817.6 g, 45% SHP to 18.7 g, and 15% NaPS to 80.0 g; that 363.4 g of 40% HAPS was added dropwise from 0 to 30 minutes, and the remaining 454.2 g thereof was added dropwise from 30 to 140 minutes; and that 34.2 of 15% NaPS was added dropwise from 0 to 130 minutes, and the remaining 45.8 g thereof was added dropwise from 130 to 200 minutes. Thus, an aqueous solution of a polymer (9) having a solid concentration of 46.2% and a pH of 3.8 was obtained.

<Example 10>

Reaction was carried out as in Example 1 except that the amount of initially fed pure water was changed to 324.8 g, 80% AA to 431.5 g, 40% HAPS to 737.0 g, 45% SHP to 57.4 g, and 15% NaPS to 49.2 g; that 327.5 g of 40% HAPS was added dropwise from 0 to 30 minutes, and the remaining 409.5 g thereof was added dropwise from 30 to 100 minutes; and that 21.0 g of 15% NaPS was added dropwise from 0 to 130 minutes, and the remaining 28.2 g thereof was added dropwise from 130 to 200 minutes. Thus, an aqueous solution of a polymer (10) having a solid concentration of 46.0% and a pH of 3.7 was obtained.

<Comparative Example 1>

A comparative polymer (1) was obtained by the following method described in Example 7 of JP H06-263803 A.

A SUS-316 stainless steel separable flask was charged with 140 g of pure water, the contents were heated to 100°C, and the flask was purged with nitrogen. Thereafter, the following components were each added dropwise into the flask through separate drip nozzles over two hours: 470 g of 80% AA, 189.5 g of a 37% aqueous solution of sodium acrylate, 813 g of 40% HAPS, 33.5 g of 35% NaPS, and 16.7 g of 45% SHP. After the dropwise addition, aging was performed for 10 minutes. Thus, an aqueous solution of the comparative polymer (1) having a solid concentration of 52.7% and a pH of 4.0 was obtained.

<Comparative Example 2>

A 2.5-L stainless steel separable flask equipped with a stirrer, a reflux condenser, and a thermometer was initially charged with 498.3 g of pure water, and the contents were heated to the boiling point under stirring. Subsequently, under stirring, the following components were each separately added dropwise into the system under reflux conditions at the boiling point: 654.7 g of 80% AA, 208.6 g of 40% HAPS, 136.1 g of 45% SHP, and 102.1 g of 15% NaPS. The dropping times were 180 minutes for 80% AA, 140 minutes for 40% HAPS, 180 minutes for 45% SHP, and 200 minutes for 15% NaPS. As for 40% HAPS, 41.7 g thereof was added dropwise continuously at a constant drop rate from 0 to 15 minutes, and the remaining 166.9 g thereof was added dropwise continuously at a constant drop rate from 15 to 140 minutes. As for 15% NaPS, 56.2 g thereof was added dropwise continuously at a constant drop rate from 0 to 130 minutes, and the remaining 45.9 g thereof was added dropwise continuously at a constant drop rate from 130 to 200 minutes. As for 80% AA and 45% SHP, they were each added dropwise continuously while the drop rate was kept constant over a period of dropwise addition. After completion of the dropwise addition of all the components, the reflux conditions at the boiling point were maintained for 30 minutes to complete the polymerization. Thus, an aqueous solution of a comparative polymer (2) having a solid concentration of 45.1% and a pH of 2.5 was obtained.

<Comparative Example 3>

A 2.5-L SUS316 stainless steel separable flask equipped with a thermometer, a reflux condenser, and a stirrer was charged with 146.5 g of pure water and 5.6 g of a 0.6% aqueous solution of Mohr's salt, and the contents were heated to 87°C under stirring (initial feeding). Subsequently, under stirring, the following components were each added dropwise into the copolymerization reaction system at a constant temperature of 87°C through separate drip nozzles: 400.7 g of 80% AA, 684.3 g of 40% HAPS, 228.3 g of 15% NaPS, 130.5 g of a 35% aqueous solution of sodium bisulfite (hereinafter, abbreviated as 35% SBS), and 4.1 g of a 35% aqueous solution of hydrogen peroxide (hereinafter, abbreviated as 35% HP). The dropping times were 180 minutes for 80% AA, 140 minutes for 40% HAPS, 170 minutes for 35% SBS, 200 minutes for 15% NaPS, and 5 minutes for 35% HP. As for the start time of dropwise addition, the components other than 35% HP were all started to be added dropwise at the same time. The component 35% HP was added dropwise in one portion 185 minutes after the start of the dropwise addition of the other components. As for 40% HAPS, 205.3 g thereof was added dropwise continuously at a constant drop rate from 0 to 30 minutes, and the remaining 479 g thereof was added dropwise continuously at a constant drop rate from 30 to 140 minutes. As for 15% NaPS, 108.4 g thereof was added dropwise continuously at a constant drop rate from 0 to 130 minutes, and the remaining 119.9 g thereof was added dropwise continuously at a constant drop rate from 130 to 200 minutes. As for 80% AA and 35% SBS, they were each added dropwise continuously while the drop rate was kept constant over a period of dropwise addition. After completion of the dropwise addition of all the components, the reflux conditions at the boiling point were maintained for 60 minutes to complete the polymerization. Thus, an aqueous solution of a comparative polymer (3) having a solid concentration of 46.3% and a pH of 3.4 was obtained.

<Comparative Example 4>

Reaction was carried out as in Comparative Example 3 except that the amount of pure water was changed to 239.5 g, 80% AA to 442.8 g, 40% HAPS to 670.4 g, 15% NaPS to 164.0 g, 35% SBS to 79.1 g, 35% HP to 4.4 g; that 167.6 g of 40% HAPS was added dropwise from 0 to 20 minutes, and the remaining 502.8 g thereof was added dropwise from 20 to 140 minutes; and that 77.9 g of 15% NaPS was added dropwise from 0 to 130 minutes, and the remaining 86.1 g thereof was added dropwise from 130 to 200 minutes. Thus, an aqueous solution of a comparative polymer (4) having a solid concentration of 45.8% and a pH of 3.2 was obtained.

<Comparative Example 5>

Reaction was carried out as in Comparative Example 2 except that the amount of initially fed pure water was changed to 534.3 g, 80% AA to 709.6 g, 40% HAPS to 226.2 g, 45% SHP to 19.4 g, and 15% NaPS to 110.7 g; that 100.6 g of 40% HAPS was added dropwise from 0 to 30 minutes, and the remaining 125.6 g thereof was added dropwise from 30 to 140 minutes; and that 47.3 g of 15% NaPS was added dropwise from 0 to 130 minutes, and the remaining 63.4 g thereof was added dropwise from 130 to 200 minutes. Thus, an aqueous solution of a comparative polymer (5) having a solid concentration of 45.9% and a pH of 2.3 was obtained.

<Comparative Example 6>

Reaction was carried out as in Comparative Example 3 except that the amount of pure water was changed to 206.2 g, 80% AA to 552.9 g, 40% HAPS to 413.8 g, 15% NaPS to 368.2 g, 35% SBS to 51.0 g, 35% HP to 2.4 g; that 124.1 g of 40% HAPS was added dropwise from 0 to 30 minutes, and the remaining 289.7 g thereof was added dropwise from 30 to 140 minutes; and that 157.4 g of 15% NaPS was added dropwise from 0 to 130 minutes, and the remaining 210.8 g thereof was added dropwise from 130 to 200 minutes. Thus, an aqueous solution of a comparative polymer (6) having a solid concentration of 45.1% and a pH of 1.8 was obtained.

The obtained polymers (1) to (10) and comparative polymers (1) to (6) were subjected to measurements of the weight average molecular weight, solid content, concentration of sulfur-containing inorganic component not incorporated into the copolymer in terms of sulfur atoms, percentage of calcium phosphate scale inhibition, and chelating ability (calcium ion trapping ability), and evaluation by the test for stability at low temperatures, by the above-described methods. The results are shown in Table 1.

[Table 1]

| | Proportion of structural unit (b) in copolymer (mol%) | | Weight average molecular weight of copolymer | Concentration of sulfur-containing inorganic component not incorporated into copolymer in terms of sulfur (ppm) | Calcium phosphate scale inhibition ability | Stability at low temperatures | Calcium ion trapping ability (mg $CaCO_3$/g) |
|---|---|---|---|---|---|---|---|
| | Amount fed | Amount analyzed | | | | | |
| Example (1) | 18.0 | 17.9 | 9000 | 3504 | 81% | Good | 180 |
| Example (2) | 24.0 | 23.6 | 5500 | 3577 | 95% | Good | 140 |
| Example (3) | 22.0 | 21.7 | 8100 | 3638 | 94% | Good | 150 |
| Example (4) | 22.0 | 21.8 | 12600 | 3649 | 90% | Good | 150 |
| Example (5) | 30.0 | 29.0 | 6000 | 2977 | 101% | Good | 70 |
| Example (6) | 7.5 | 7.5 | 6000 | 1758 | 43% | Good | 230 |
| Example (7) | 11.0 | 10.9 | 6900 | 1500 | 76% | Good | 290 |
| Example (8) | 14.0 | 13.8 | 15500 | 1628 | 56% | Good | 220 |
| Example (9) | 25.0 | 24.1 | 14700 | 1686 | 96% | Good | 200 |
| Example (10) | 22.0 | 21.3 | 5500 | 1265 | 99% | Good | 170 |
| Comparative Example (1) | 20.0 | 17.7 | 19000 | 1992 | 24% | Good | 160 |
| Comparative Example (2) | 5.0 | 5.0 | 3000 | 2152 | 36% | Good | 210 |
| Comparative Example (3) | 22.0 | 21.3 | 4600 | 6049 | 73% | Poor | 130 |
| Comparative Example (4) | 18.0 | 17.3 | 10500 | 8818 | 91% | Poor | 120 |
| Comparative Example (5) | 5.0 | 5.0 | 35900 | 1923 | 2% | Good | 300 |
| Comparative Example (6) | 11.0 | 11.0 | 12200 | 9219 | 14% | Poor | 220 |

The evaluation results demonstrated that the (meth)acrylic acid copolymer-containing composition of the present invention has a better calcium phosphate scale inhibition ability and better stability at low temperatures than conventional polymer compositions.

## Claims

1. A (meth)acrylic acid copolymer-containing composition comprising a (meth)acrylic acid copolymer including:

   a structural unit (a) derived from at least one selected from the group consisting of (meth)acrylic acid and salts thereof; and
   a structural unit (b) derived from a sulfonic acid monomer (B),
   the copolymer containing the structural unit (b) in a proportion of 5 to 90 mol% based on 100 mol% of all structural units, and having a weight average molecular weight of 4,500 to 18,000,
   the composition containing a sulfur-containing inorganic component not incorporated into the copolymer at a concentration of 6,000 ppm or lower in terms of sulfur atoms.

2. The (meth)acrylic acid copolymer-containing composition according to claim 1,
   wherein the copolymer contains the structural unit (b) in a proportion of 10 to 90 mol% based on 100 mol% of all structural units.

3. The (meth)acrylic acid copolymer-containing composition according to claim 1 or 2,
   wherein the copolymer has a weight average molecular weight of 4,500 to 17,000.

4. The (meth)acrylic acid copolymer-containing composition according to any one of claims 1 to 3,
   wherein the sulfonic acid monomer (B) is represented by the following formula (1):

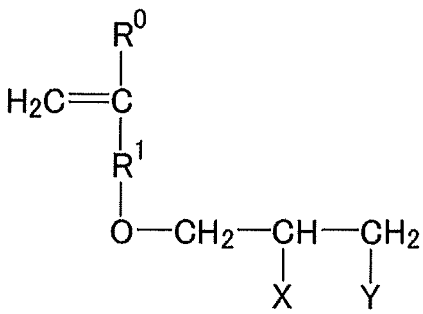

(1)

   wherein $R^0$ is a hydrogen atom or a methyl group; $R^1$ is a $CH_2$ group, a $CH_2CH_2$ group, or a direct bond; X and Y are each selected from the group consisting of a hydroxy group and structures represented by the following formulas (2) and (3), with at least one of X or Y being a structure represented by the formula (2) or (3):

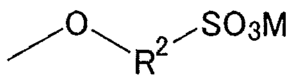

(2)

$-SO_3M$ (3)

   wherein $R^2$ is an optionally substituted C1-C10 alkylene group or an optionally substituted C6-C10 arylene group; M in the formulas (2) and (3) is at least one selected from the group consisting of a hydrogen atom, a metal atom, an ammonium group, an organic amine salt, and an organic ammonium group.

5. The (meth)acrylic acid copolymer-containing composition according to any one of claims 1 to 4,
   wherein the copolymer contains a phosphorus-containing group.

6. The (meth)acrylic acid copolymer-containing composition according to any one of claims 1 to 5,
   wherein the copolymer contains at least one structure derived from one selected from the group consisting of hypophosphorous acid, salts thereof, phosphorous acid, and salts thereof.

**7.** The (meth)acrylic acid copolymer-containing composition according to any one of claims 1 to 6, wherein the composition has a percentage of scale inhibition of 40% or higher determined by a test for calcium phosphate scale inhibition, the test for calcium phosphate scale inhibition including:

allowing a test solution containing the copolymer at a concentration of 12 mg/L and phosphoric acid ions at a concentration of 9 mg $PO_4^{3-}$/L and having a calcium hardness in terms of calcium carbonate of 500 mg $CaCO_3$/L to stand at 60°C for 24 hours; and
analyzing a concentration of residual phosphoric acid ions to determine a percentage of calcium phosphate scale inhibition using the following formula:

$$\text{Percentage of calcium phosphate scale inhibition (\%)} = 100 \times (R - Q)/(P - Q)$$

wherein P is a concentration (mg/L) of phosphoric acid ions in the test solution at the start of the test (prepared solution); Q is a concentration (mg/L) of residual phosphoric acid ions in a blank solution not containing the copolymer; and R is a concentration (mg/L) of residual phosphoric acid ions.

**8.** A water treatment chemical comprising the (meth)acrylic acid copolymer-containing composition according to any one of claims 1 to 7.

**9.** A cleaning agent comprising the (meth)acrylic acid copolymer-containing composition according to any one of claims 1 to 7.

**10.** A method of producing a (meth)acrylic acid copolymer, the method comprising:

polymerizing a monomer component containing at least one selected from the group consisting of (meth)acrylic acid and salts thereof and a sulfonic acid monomer (B) in the presence of a phosphorus-containing compound to prepare a copolymer,
the monomer component containing the sulfonic acid monomer (B) in a proportion of 5 to 90 mol% based on 100 mol% of the entire monomer component,
the copolymer having a weight average molecular weight of 4,500 to 18,000.

**11.** The method of producing a (meth)acrylic acid copolymer according to claim 10, wherein the copolymer contains a structural unit (b) in a proportion of 10 to 90 mol% based on 100 mol% of all structural units.

**12.** The method of producing a (meth)acrylic acid copolymer according to claim 10 or 11, wherein the copolymer has a weight average molecular weight of 4,500 to 17,000.

**13.** The method of producing a (meth)acrylic acid copolymer according to any one of claims 10 to 12, wherein in the polymerization, the sulfonic acid monomer (B) is continuously added dropwise to a reaction vessel at two or more different drop rates.

**14.** The method of producing a (meth)acrylic acid copolymer according to any one of claims 10 to 13, wherein the phosphorus-containing compound is hypophosphorous acid or a salt thereof.

## INTERNATIONAL SEARCH REPORT

International application No.
PCT/JP2019/009053

A. CLASSIFICATION OF SUBJECT MATTER
Int.Cl. C08L33/02(2006.01)i, A61K8/81(2006.01)i, A61Q5/02(2006.01)i, A61Q11/00(2006.01)i, A61Q19/10(2006.01)i, C08F2/38(2006.01)i, C08F220/06(2006.01)i, C08F228/02(2006.01)i, C08K3/30(2006.01)i, C11D3/37(2006.01)i

According to International Patent Classification (IPC) or to both national classification and IPC

B. FIELDS SEARCHED

Minimum documentation searched (classification system followed by classification symbols)
Int.Cl. C08L33/02, A61K8/81, A61Q5/02, A61Q11/00, A61Q19/10, C08F2/38, C08F220/06, C08F228/02, C08K3/30, C11D3/37

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2015-193529 A (NIPPON SHOKUBAI CO., LTD.) 05 November 2015, claims, examples & WO 2015/141667 A1 | 1-6, 10-14<br>7-9 |
| Y | JP 2016-176029 A (NIPPON SHOKUBAI CO., LTD.) 06 October 2016, claims, paragraph [0001] (Family: none) | 7-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 May 2019 (14.05.2019) | 28 May 2019 (28.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/009053

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-217899 A (NIPPON SHOKUBAI CO., LTD.) 05 August 2004, claims, paragraph [0004] & US 2004/0127660 A1, claims, paragraph [0004] & EP 1433796 A1 | 7-9 |
| Y | JP 2013-212435 A (KURITA WATER INDUSTRIES LTD., NIPPON SHOKUBAI CO., LTD.) 17 October 2013, claims, paragraph [0074], examples & WO 2013/147112 A1 & TW 201348150 A & CN 104203842 A | 7-8 |
| A | JP 2015-25160 A (KURITA WATER INDUSTRIES LTD., NIPPON SHOKUBAI CO., LTD.) 05 February 2015 & US 2016/0167997 A1 & WO 2015/012362 A1 & TW 201512109 A & CN 105408519 A & SG 11201600454Y A | 1-14 |
| A | JP 2011-72851 A (NIPPON SHOKUBAI CO., LTD.) 14 April 2011 (Family: none) | 1-14 |
| A | JP 2017-214495 A (NIPPON SHOKUBAI CO., LTD.) 07 December 2017 (Family: none) | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002003536 A
- JP H06263803 A
- JP H06287208 A
- JP 2014511402 T